(19) Europäisches Patentamt
European Patent Office
Office européen des brevets

(11) Publication number: 0 134 928 B1

(12) # EUROPEAN PATENT SPECIFICATION

(45) Date of publication of patent specification: 03.02.88

(21) Application number: 84107134.3

(22) Date of filing: 20.06.84

(51) Int. Cl.[4]: C 07 D 487/04, A 61 K 31/505, A 61 K 31/415

(54) ## Imidazo1,5-arpyrimidine derivatives and process for their preparation.

(30) Priority: 12.07.83 JP 126697/83

(43) Date of publication of application: 27.03.85 Bulletin 85/13

(45) Publication of the grant of the patent: 03.02.88 Bulletin 88/05

(84) Designated Contracting States: BE CH DE FR GB IT LI NL SE

(56) References cited:
DE-A-2 160 020
US-A-4 236 005

BULLETIN DE LA SOCIETE CHIMIQUE DE FRANCE, 1972 P. GUERRET et al. "Recherches dans la série des azoles. XCI. - Réactivité de l'imidazo (1,5-a) pyrimidine" pages 2481-2483

BULLETIN DE LA SOCIETE CHIMIQUE DE FRANCE, 1971 P. GUERRET et al. "Recherches dans la série des azoles. LXXIX. - Imidazo (1,5-a) pyrimidines: Synthèse et étude spectroscopique" pages 1031-1037

(73) Proprietor: KYORIN PHARMACEUTICAL CO., LTD.
No. 5, Kanda Surugadai 2-chome
Chiyoda-ku Tokyo (JP)

(72) Inventor: Irikura, Tsutomu
No. 10-28, Ooizumigakuen-cho 7-chome
Nerima-ku Tokyo (JP)
Inventor: Suzue, Seigo
No. 13-4, Aoba 4-chome
Kuki-shi Saitama-ken (JP)
Inventor: Uchida, Hiroaki
No. 22-12, Kamiuma 4-chome
Setagaya-ku Tokyo (JP)
Inventor: Shinoda, Hirotaka
No. 3-1, Kamiaoki 1-chome
Kawaguchi-shi Saitama-ken (JP)
Inventor: Murayama, Satoshi
No. 6095, Ooaza Tomonuma Nogi-cho
Shimotsuga-gun Tochigi-ken (JP)
Inventor: Kinoshita, Susumu
No. 5932, Ooaza Tomonuma Unoki Nogi-cho
Shimotsuga-gun Tochigi-ken (JP)

(74) Representative: Patentanwälte TER MEER - MÜLLER - STEINMEISTER
Mauerkircherstrasse 45
D-8000 München 80 (DE)

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

EP 0 134 928 B1

Courier Press, Leamington Spa, England.

**Description**

This invention relates to a new class of 2,4-dichloro-imidazo[1,5-a]pyrimidine derivatives, salts of these compounds, such as the hydrochloride, sulfate, acetate, tartrate and methansulfonate, a process for their preparation and pharmaceutical compositions comprising these compounds.

Recently, fungal diseases are on the increase internationally because of frequently using broad spectrum antibiotics, steroid hormones and immunosuppressive agents et cetera. However, useful antifungal agents for the therapy of fungal diseases are rare. At present, it might almost be said that the drugs for fungal diseases are polyenmacrolide and imidazole derivatives. It has been expected to develop more useful antifungal agents for treatment of fungal diseases.

Bull. Soc. Chim. de France, 9172, 1031—1037 and 2481—2483 disclose imidazo[1,5-a]pyrimidines without describing any biological activity thereof.

US—A—4 236 005 describes imidazo[1,5-a]pyrimidines comprising an aryl substituent on the six-membered ring and being described as having anxiolytic activity.

DE—A—2 160 020 covers perchloro-imidazopyrimidine, which is disclosed to have a fungicidal activity for plants only and a good compatibility with warm blooded animals.

It has now been found that the novel imidazol[1,5-a]pyrimidine derivatives with a different structure have a high potency against many different organisms as compared with known antifungal activities, which may not only be used as a medicine for human but also as a drug for animal, fish, shells and antiseptic for food in the various forms.

The subject-matter of the invention therefore relates to 2,4-dichloroimidazo[1,5-a]pyrimidine derivatives having the following formula (I)

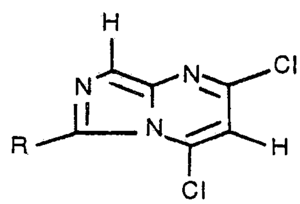

I

wherein R is selected from the group of substituents comprising 4-chlorophenyl, 4-bromophenyl, 3,4-dichlorophenyl, 2,4-dichlorophenyl, 4-chlorobenzyl, 1-phenyl-ethyl, 2,4-dichlorobenzyl, 4-iodophenyl and cyclohexyl groups, and the acid addition salts thereof.

As suitable salts of the compounds represented by the formula (I), there may be mentioned salts derived from inorganic acids, such as, for example, hydrochloric acid, sulfuric acid, or salts derived from organic acids, such as, for example, acetic acid, tartaric acid or methanesulfonic acid.

These new compounds can be prepared by the method mentioned below.

The α-acylaminoalkylpyrimidine compounds represented by the formula (II) are converted to the imidazo[1,5-a]pyrimidine compounds having the formula (III) by the condensing agents such as phosphoryl chloride and thionyl chloride:

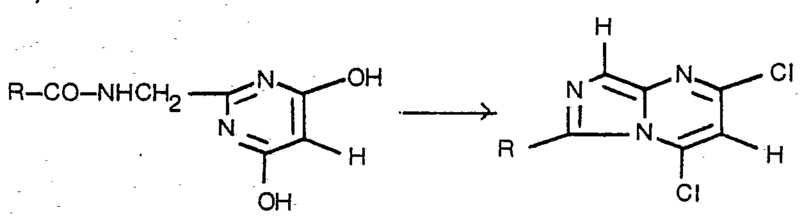

II I

wherein $R_2$ has the previously defined meanings.

α-Acylaminoalkylpyrimidine compounds having the formula (II), the starting materials in the reaction discussed above, are also novel compounds and can be prepared by two methods. One method of preparation of these compounds involves (a) conversion of the α-aminoacetonitrile to the N-acyl derivatives of aminoacetonitrile using various acid chloride, (b) treatment of the N-acyl derivatives with hydrogen chloride in ethanol followed by treatment with ammonia in ethanol to give the amidine derivatives and (c) reaction of the latter compounds with diethyl malonate to give the desired compound (II). These reactions are summarized in the following scheme.

1) $HCl/C_2H_5OH$
2) $NH_3/C_2H_5OH$

$$R—COCl + NH_2—CH_2—CN \xrightarrow{(a)} R—CONHCH_2—CN \xrightarrow[(b)]{} R—CONH—CH_2—\overset{\overset{NH}{\|}}{C}—NH_2 \xrightarrow[(c)]{CH_2 = (COOC_2H_5)_2}$$

(II)

wherein R is as above.

An alternative method for preparing these compounds involves (d) reactions of glycine ethyl ester hydrochloride with various acid chlorides to give N-acyl derivatives of glycine ethyl ester and (e) treatment of these N-acyl derivatives with malonamide to give the desired compounds (II). These reactions are summarized in the following scheme:

$$R{-}COCl + NH_2{-}CH_2COOC_2H_5 \cdot HCl \xrightarrow{(d)} R{-}CONHCH_2COOC_2H_5 \xrightarrow[(e)]{CH_2=(CONH_2)_2} \quad (II)$$

wherein R is as above.

Preparation of intermediate compounds:

Example A

Preparation of 2-(3,4-dichlorobenzylaminomethyl)-4,6-dihydroxypyrimidine

(i) The stirred solution of aminoacetonitrile sulfate (42 g) in water (180 ml) is cooled in an ice bath and the solution of sodium carbonate (51 g) in water (250 ml) is added in portions.

Then, 3,4-dichlorobenzylchloride (63 g) is added and the mixture is stirred vigorously for 5 hours at room temperature.

Filtration followed by recrystallization from ethanol give acyl derivative (53.9 g).

(ii) Part (i) (53.9 g) is added to the solution of hydrogen chloride gas (68.6 g) in ethanol (500 ml) the mixture is stirred vigorously in an ice bath for 17 hours. Ethanol is evaporated, the residue is poured into ice-water, neutralized by sodium bicarbonate solution, and extracted with chloroform. The chloroform layer is washed with water, dried over sodium sulfate and evaporated to give yellow crystal (50 g).

(iii) Part (ii) (50 g) is added to the ice cooled solution of ammonia gas (247 g) in ethanol (300 ml) and the mixture is stirred for 14 hours. The precipitated crystal is filtered to give amide derivatives (38.5 g).

(iv) Part (iii) is added to the solution of sodium (5.6 g) in ethanol (250 ml) then diethyl malonate (13 g) is added and the mixture is stirred for 8 hours at 60°C. Ethanol is evaporated, the residue is dissolved in water and washed with ether. The water layer is neutralized with acetic acid. Precipitate is filtered, washed with water and recrystallized from dimethylformamide to give 2-(3,4-dichlorobenzoylaminomethyl)-4,6-dihydroxypyrimidine (11.4 g) as colorless crystal. m.p. 268—270°C (dec.).

| | | | |
|---|---|---|---|
| Anal. (%) Calcd. for $C_{12}H_9N_3O_3Cl_2$: | C, 45.88; | H, 2.89; | N, 13.38 |
| Found: | C, 45.97; | H, 2.76; | N, 13.44 |

Other compounds prepared by the method of this example are as follows.

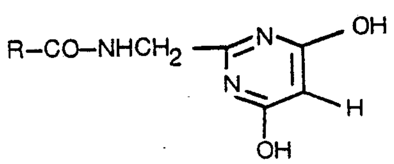

| R | m.p. (°C) |
|---|---|
| 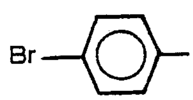 | >300 |
| 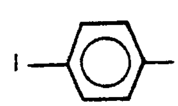 | >300 |
| 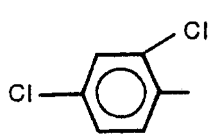 | 263—269 (dec.) |
| 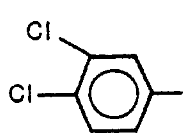 | — |

## Example B

Preparation of 2-(4-chlorobenzylcarbonylaminomethyl)-4,6-dihydroxypyrimidine

(i) The mixture of the solution of glycin ethyl ester hydrochloride (38.1 g) and potassium carbonate (150 g) in water (700 ml), benzene (600 ml) and ether (450 ml) is stirred vigorously at room temperature. The solution of 4-chlorophenylacetylchloride (61.5 g) in benzene (100 ml) is added dropwise to above mixture during 30 minutes and stirred for 3 hours. The organic layer is dried over sodium sulfate, evaporated to 200 ml and cooled. Precipitate is filtered to give acyl derivative of glycin ethyl ester (44.1 g) as colorless crystal.

(ii) Malonamide (9.5 g) is added to the solution of sodium (4.04 g) in ethanol (400 ml) and stirred at 50°C for 1 hour.

Part (i) is added to the mixture and refluxed for 6 hours. Ethanol is evaporated, the residue is dissolved in water and neutralized with acetic acid. Precipitate is filtered, washed with water and recrystallized from dimethylformamide to give 2-(4-chlorobenzylcarbonylaminomethyl)-4,6-dihydroxypyrimidine (9 g) as colorless crystal. m.p. 280—283°C (dec.)

| | | | |
|---|---|---|---|
| Anal. (%) Calcd. for $C_{13}H_{12}N_3O_3Cl$: | C, 53.16; | H, 4.12; | N, 14.31 |
| Found: | C, 53.11; | H, 4.07; | N, 14.51 |

The following compound can be prepared by the method of this example.

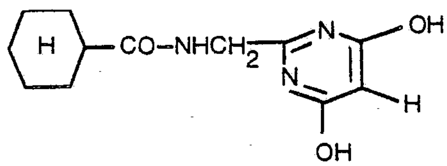

m.p. 260—265°C (dec.)

## Experiment 1

The antifungal activity of the compound of the present invention was assayed by the standard agar dilution streak method against fungi. The results were shown in Table 1.

M.I.C. studies with representative members of the compound of this invention have demonstrated extremely favorable antimycotic activity, so that these compounds will be very useful as therapeutic agents, drugs for animals, fishes and shells, and an antiseptic for food.

TABLE 1

Antifungal Activity

| Organismus | Minimum Inhibitory Concentration (µg/ml) | | | | | | | | |
|---|---|---|---|---|---|---|---|---|---|
| | Ex. 1 | Ex. 2 | Ex. 3 | Ex. 4 | Ex. 5 | Ex. 6 | Ex. 7 | Ex. 8 | Ex. 9 |
| Candida albicans 3147 | 6.25 | 1.56 | 1.56 | 1.56 | 1.56 | 3.13 | 1.56 | 0.78 | 1.56 |
| Candida albicans IFO—1388 | 1.56 | 1.56 | 1.56 | 0.78 | 1.56 | 0.78 | 0.20 | 0.78 | 3.13 |
| Candida albicans IFO—1594 | 3.13 | 3.13 | 1.56 | 3.13 | 1.56 | 3.13 | 0.39 | 0.78 | 6.25 |
| Candida albicans MTU—12124 | 3.13 | 3.13 | 1.56 | 1.56 | 6.25 | 3.13 | 6.25 | 0.78 | 1.56 |
| Candida albicans KYF—602 | 6.25 | 6.25 | 3.13 | 6.25 | 3.13 | 12.5 | 3.13 | >6.25 | >6.25 |
| Candida stellatidea IFO—1398 | 0.20 | 0.20 | 0.39 | 0.78 | 0.78 | 3.13 | 3.13 | 0.20 | 1.56 |
| Microsporum canis 20010 | 0.78 | 0.78 | 0.39 | 1.56 | 0.39 | 1.56 | 1.56 | 0.78 | |
| Aspergillus fumigatus MTU—06002 | 6.25 | 6.25 | >100 | 12.5 | 3.13 | 25 | 50 | >6.25 | |
| Trichophyton mentagrophytes MTU—19003 | 1.56 | 1.56 | 0.78 | 1.56 | 1.56 | 1.56 | 3.13 | 1.56 | |
| Trichophyton mentagrophytes MTU—19005 | 0.78 | 0.78 | 0139 | 0.78 | 0.20 | 1.56 | 1.56 | 0.78 | |

The following examples are further illustrative of this invention.

Preparation of compounds of this invention.

## Example 1

Preparation of 2,4-dichloro-6-(4-chlorophenyl)imidazo[1.5-a]pyrimidine

2-(4-Chlorobenzoylaminomethyl)-4,6-dihydroxypyrimidine (1 g) is added to phosphoryl chloride (10 ml) and refluxed for 3 hours. Excess phosphoryl chloride is evaporated in vacuo, the residue is neutralized with sodium bicarbonate aqueous solution and extracted with chloroform. The chloroform layer is washed with water, dried over sodium sulfate and evaporated to give yellowish-brown residue. Purification by alumina column-chromtography (eluted with benzene) followed by recrystallization from ethanol give 2,4-dichloro-6-(4-chlorophenyl)imidazo[1,5-a]pyrimidine (0.34 g) as yellow prisms. m.p. 203—205°C.

Anal. (%) Calcd. for $C_{12}H_6N_3Cl_3$: C, 48.28; H, 2.03; N, 14.07
Found: C, 48.30; H, 1.85; N, 14.05

Other compounds prepared by the method of Example 1 are as follows.

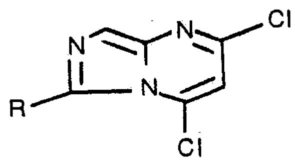

| Ex. No. | R | m.p. (°C) | Molecular Formula | Anal. (%); Calcd. (Found) C | H | N |
|---|---|---|---|---|---|---|
| 2 | 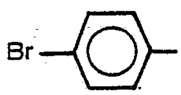 | 202—203 | $C_{12}H_6N_3BrCl_2$ | 42.02(42.03) | 1.76(1.56) | 12.25(12.27) |
| 3 | 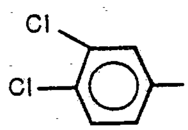 | 188—189 | $C_{12}H_5N_3Cl_4$ | 43.28(43.39) | 1.51(1.33) | 12.62(12.72) |
| 4 | 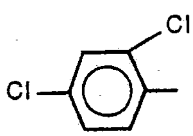 | 155—156 | $C_{12}H_5N_3Cl_4$ | 43.28(43.53) | 1.51(1.48) | 12.62(12.52) |

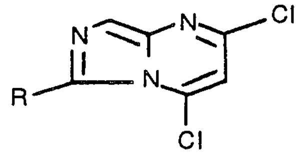

| Ex. No. | R | m.p. (°C) | Molecular Formula | Anal. (%); Calcd. (Found) C | H | N |
|---|---|---|---|---|---|---|
| 5 | 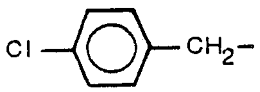 | 170—171 | $C_{13}H_8N_3Cl_3$ | 49.95(49.85) | 2.58(2.40) | 13.44(13.52) |
| 6 | 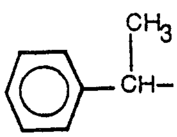 | 100—101 | $C_{14}H_{11}N_3Cl_2$ | 57.55(57.51) | 3.79(3.61) | 14.38(14.48) |
| 8 | 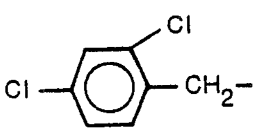 | 158—159 | $C_{13}H_7N_3Cl_4$ | 44.99(45.07) | 2.03(1.85) | 12.01(12.12) |
| 9 | 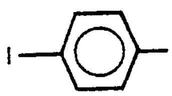 | 197—199 | $C_{12}H_6N_3Cl_2I$ | 36.96(37.07) | 1.55(1.47) | 10.77(10.78) |
| 7 | 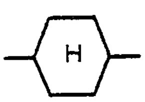 | 171—172 | $C_{12}H_{13}N_3Cl_2$ | 53.35(53.45) | 4.85(4.79) | 15.55(15.65) |

## Claims

1. 2,4-Dichloro-imidazo[1,5-a]pyrimidine derivatives having the following formula (I)

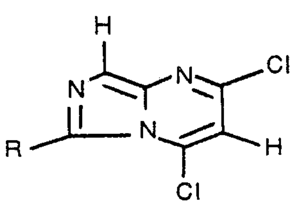

I

wherein R is selected from the group of substituents comprising 4-chlorophenyl, 4-bromophenyl, 3,4-dichlorophenyl, 2,4-dichlorophenyl, 4-chlorobenzyl, 1-phenyl-ethyl, 2,4-dichlorobenzyl, 4-iodophenyl and cyclohexyl groups, and the acid addition salts thereof.

2. A pharmaceutical composition comprising a compound according to claim 1 together with a pharmaceutically acceptable carrier.

3. The compounds of claim 1 for use as antimycotic agents.

4. A process for preparing the compounds according to claim 1, characterized by converting a compound having the formula (II)

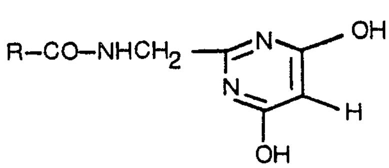

II

wherein R has the definitions provided in claim 1, with a condensing agent, such as phosphorylchloride or thionylchlorid.

## Patentansprüche

1. 2,4-Dichlorimidazo[1,5-a]pyrimidinderivate der Formel (I)

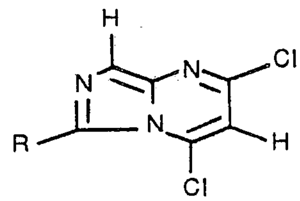

I

worin R aus der Gruppe von Substituenten der 4-Chlorphenyl-, 4-Bromphenyl-, 3,4-Dichlorphenyl-, 2,4-Dichlorphenyl-, 4-Chlorbenzyl-, 1-Phenylethyl-, 2,4-Dichlorbenzyl, 4-Jodphenyl- und Cyclohexylgruppen umfassenden Gruppe gewählt ist, und deren Säureadditionssalze.

2. Pharmazeutische Zusammensetzung enthaltend eine Verbindung nach Anspruch 1 und eine pharmazeutisch annehmbare Trägersubstanz.

3. Verbindungen nach Anspruch 1 zur Verwendung als antimycotische Mittel.

4. Verfahren zur Herstellung der Verbindungen nach Anspruch 1, dadurch gekennzeichnet, daß man eine Verbindung der Formel (II)

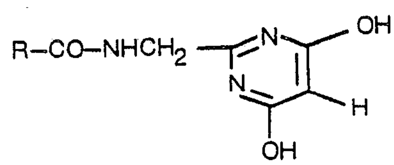

II

worin R die in Anspruch 1 angegebenen Bedeutungen besitzt, mit einem Kondensationsmittel, wie Phosphorylchlorid oder Thionylchlorid, unwandelt.

## Revendications

1. Dérivés de la 2,4-dichloro-imidazo(1,5-a)pyrimidine ayant la formule suivante (I)

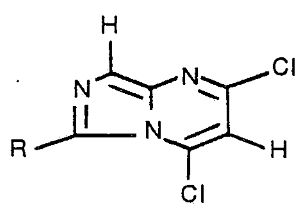

I

dans laquelle R est choisi dans le groupe de substituants comprenant les groupes 4-chlorophényle, 4-bromophényle, 3,4-dichlorophényle, 2,4-dichlorophényle, 4-chlorobenzyle, 1-phényl-éthyle, 2,4-dichlorobenzyle, 4-iodophényle et cyclohexyle, et les sels d'addition de ceux-ci avec un acide.

2. Composition pharmaceutique comprenant un composé selon la revendication 1 aussi qu'un véhicule pharmaceutiquement acceptable.

3. Composés de la revendication 1 destiné à être utilisé comme agents antimycosiques.

4. Procédé de préparation des composés selon la revendication 1 caractérisé en ce que l'on convertit un composé ayant la formule (II)

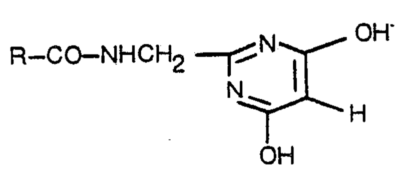

II

dans laquelle R a les définitions données dans la revendication 1 avec un agent de condensation comme le chlorure de phosphoryle ou le chlorure de thionyle.